# EUROPEAN PATENT SPECIFICATION

(11) **EP 3 100 711 B1**
(45) Date of publication and mention of the grant of the patent: **29.08.2018**
(21) Application number: 15743672.6
(22) Date of filing: 27.01.2015
(51) Int. Cl.: A61J 3/00, A61J 1/20, A61M 5/142, A61M 5/145

(54) **LIQUID MEDICINE FILLING UNIT, LIQUID MEDICINE RESERVOIR INSTRUMENT, AND FILLING ADAPTER**
FLÜSSIGMEDIKAMENTFÜLLEINHEIT, RESERVOIRINSTRUMENT FÜR FLÜSSIGMEDIKAMENT UND FÜLLADAPTER
UNITÉ DE REMPLISSAGE DE MÉDICAMENT LIQUIDE, INSTRUMENT À RÉSERVOIR DE MÉDICAMENT LIQUIDE, ET ADAPTATEUR DE REMPLISSAGE

(30) Priority: 30.01.2014 JP 2014015114
(43) Date of publication of application: 07.12.2016
(73) Proprietor: Terumo Kabushiki Kaisha, Tokyo 151-0072 (JP)
(72) Inventor: UCHIYAMA, Joji, Ashigarakami-gun Kanagawa 259-0151 (JP)
(74) Representative: Casalonga
(86) International application number: PCT/JP2015/052217
(87) International publication number: WO 2015/115435

(56) References cited:
- JP-A- H0 280 048
- JP-A- H08 308 925
- JP-A- 2004 275 465
- JP-A- 2004 329 685
- JP-A- 2010 501 283

## Description

### Technical Field

The present invention is defined in claim 1, and relates to a drug solution filling unit that fills, with a drug solution, a drug solution reservoir part of a portable drug solution administration device such as, for example, an insulin pump, and to a drug solution reservoir instrument and a filling adapter constituting the drug solution filling unit. The present invention relates to a drug solution filling unit according to the preambe of claim 1, such as it is, e.g., known from JPH02-80048.

### Background Art

In recent years, a treatment method to continually administer a drug solution into a body of a patient by means of a hypodermic injection, an intravenous injection or the like has been performed. For example, as a treatment method for a diabetic patient, a treatment to continually administer a tiny amount of insulin into a body of a patient has been performed. Since a drug solution (insulin) is administered to a patient all day in this treatment method, a portable drug solution administration device (so-called insulin pump) that is fixed to a body or clothes of a patient and enabled to be carried is used.

An example of a conventional drug solution administration device includes a drug solution administration device described in Patent Literature 1. The drug solution administration device described in Patent Literature 1 has a drug solution reservoir part that reserves a drug solution and a piston that is driven in the drug solution reservoir part. The drug solution is pushed out of the drug solution reservoir part by the piston, whereby the drug solution is administered to a user.

By the time the drug solution administration device is delivered to the user, however, the drug solution reservoir part generally has not been filled with the drug solution in advance. When using the drug solution administration device, therefore, the user needs to perform work of filling the drug solution reservoir part from a separate drug solution container (for example, vial) in which the drug solution is reserved.

In a conventional method for filling a drug solution reservoir part, for example, a user first extracts a drug solution from a drug solution container using a syringe. The method then causes a needle tube of the syringe to puncture a filling port provided on the drug solution reservoir part, whereby the drug solution reservoir part is filled with the drug solution.

In another conventional method for filling a drug solution reservoir part, for example, the drug solution reservoir part and a drug solution container are connected first using an adapter. The method next causes a pusher provided on the drug solution reservoir part to be pulled, whereby the drug solution reservoir part is filled with a drug solution in the drug solution container.

### Citation List

### Patent Literature

Patent Literature 1: JP 2010-501283 W

### Summary of Invention

### Technical Problem

The above-mentioned filling method that uses a syringe, however, has had such a problem that a needle tube of the syringe is exposed during the filling work and therefore might puncture a user by mistake.

In addition, in the filling method that uses the syringe and the filling method that uses a pusher of a drug solution reservoir part, air might remain in the drug solution reservoir part when the drug solution reservoir part is filled with a drug solution from a drug solution container. In the conventional method for filling the drug solution reservoir part, therefore, a process for discharging air that remains in the drug solution reservoir part is required, which makes the filling work significantly complicated.

In consideration of the above-mentioned problems, an object of the present invention is to provide a drug solution filling unit, a drug solution reservoir instrument, and a filling adapter capable of preventing air from remaining in a drug solution reservoir part and easily filling the drug solution reservoir part with a drug solution.

### Solution to Problem

In order to solve the above-mentioned problems and achieve the object of the present invention, a drug solution filling unit of the present invention includes a drug solution reservoir instrument and a filling adapter. The drug solution reservoir instrument includes a drug solution reservoir part that reserves a drug solution and an exterior case that houses the drug solution reservoir part. The drug solution reservoir part has a filling port from which the drug solution is injected and a liquid supply port that sends the filled drug solution. The filling adapter includes an air vent needle tube, a liquid supply needle tube, and a suction channel. The air vent needle tube is connected to a drug solution container and through which gas that is sent to the drug solution container passes. The liquid supply needle tube is connected to the drug solution container and the filling port and through which the drug solution that is sent from the drug solution container to the drug solution reservoir part passes. The suction channel communicates with the liquid supply port and communicates with a suction mechanism that sucks in gas within the drug solution reservoir part.

In addition, a drug solution reservoir instrument of the present invention includes: a drug solution reservoir part having a filling port from which a drug solution is injected and a liquid supply port that sends the filled drug solution; and an exterior case that houses the drug solution reservoir part. Gas within the drug solution reservoir part is sucked from the liquid supply port when the drug solution is filled.

In addition, a filling adapter of the present invention includes a main body part, an air vent needle tube, a liquid supply needle tube, and a suction channel. The main body part is detachably mounted on a drug solution reservoir instrument having a drug solution reservoir part that reserves a drug solution. The air vent needle tube is held by the main body part, connected to a drug solution container, and through which gas that is sent to the drug solution container passes. The liquid supply needle tube is held by the main body part, connected to the drug solution container and a filling port, and through which the drug solution that is sent from the drug solution container to the drug solution reservoir part passes, the filling port being provided on the drug solution reservoir part, the drug solution being injected from the filling port. The suction channel communicates with a liquid supply port and communicates with a suction mechanism that sucks in gas within the drug solution reservoir part, the liquid supply port being provided on the drug solution reservoir part to send the drug solution.

### Advantageous Effects of Invention

According to a drug solution filling unit, a drug solution reservoir instrument, and a filling adapter of the present invention, in order to fill a drug solution reservoir part with a drug solution while sucking in air within the drug solution reservoir part, it is possible to prevent air from remaining in the drug solution reservoir part of a drug solution administration device and easily fill the drug solution reservoir part with the drug solution.

### Brief Description of Drawings

Fig. 1 is a perspective view illustrating an exemplary embodiment of a drug solution filling device and a drug solution filling unit of the present invention.
Fig. 2 is an exploded perspective view illustrating the exemplary embodiment of the drug solution filling device and the drug solution filling unit of the present invention.
Fig. 3 is an exploded perspective view illustrating the exemplary embodiment of the drug solution filling device of the present invention.
Fig. 4 is a perspective view of the exemplary embodiment of the drug solution filling device of the present invention viewed from a back side.
Fig. 5 is a front view illustrating the exemplary embodiment of the drug solution filling device and the drug solution filling unit of the present invention.
Figs. 6A and 6B are side views illustrating a filling amount adjustment mechanism in the exemplary embodiment of the drug solution filling device of the present invention, where Fig. 6A is the side view illustrating a normal state and Fig. 6B is the side view illustrating a state where a releasing button is pressed.
Fig. 7 is a perspective view illustrating the exemplary embodiment of the drug solution filling unit of the present invention.
Fig. 8 is a perspective view illustrating a principal part of a drug solution reservoir instrument in the exemplary embodiment of the drug solution filling unit of the present invention.
Fig. 9 is an exploded perspective view illustrating a filling adapter in the exemplary embodiment of the drug solution filling unit of the present invention.
Fig. 10 is a cross-sectional view illustrating the filling adapter in the exemplary embodiment of the drug solution filling unit of the present invention.
Fig. 11 is a cross-sectional view illustrating a principal part in the exemplary embodiment of the drug solution filling device and the drug solution filling unit of the present invention.
Fig. 12 is a front view illustrating operation for filling a drug solution reservoir part with a drug solution in the exemplary embodiment of the drug solution filling device and the drug solution filling unit of the present invention.
Figs. 13A and 13B are explanatory views schematically illustrating the drug solution filling operation in the exemplary embodiment of the drug solution filling device and the drug solution filling unit of the present invention.
Figs. 14A to 14D are explanatory views illustrating operation for removing the filling adapter from the drug solution reservoir instrument in the exemplary embodiment of the drug solution filling unit of the present invention.

### Description of Embodiments

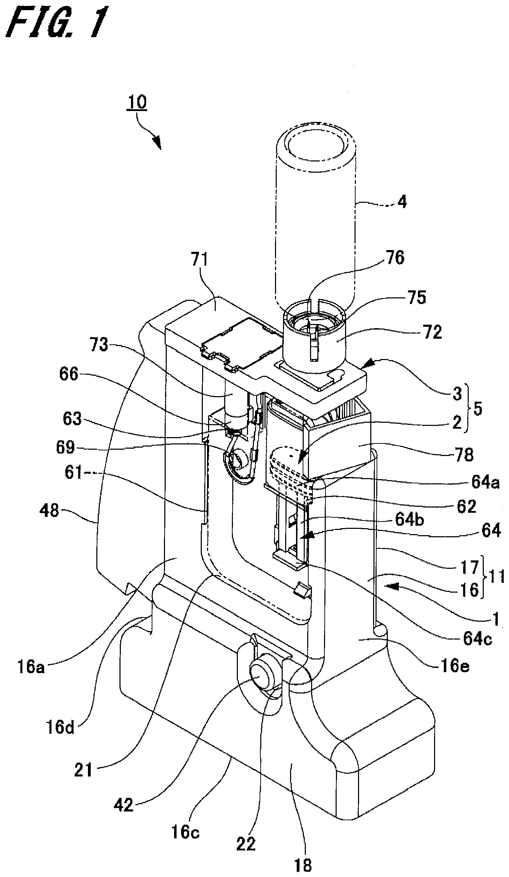

Hereinafter, an exemplary embodiment of a drug solution filling device and a drug solution filling unit of the present invention will be described with reference to Figs. 1 to 14. Members that are common to the respective drawings are denoted by the same reference signs. The present invention is not limited to the following embodiment.

### <1. Exemplary Embodiment>

### 1-1. Exemplary Configuration of Drug Solution Filling Device and Drug Solution Filling Unit

First, an exemplary embodiment (hereinafter referred to as the "present example") of a drug solution filling device and a drug solution filling unit of the present invention will be described with reference to Figs. 1 to 10.

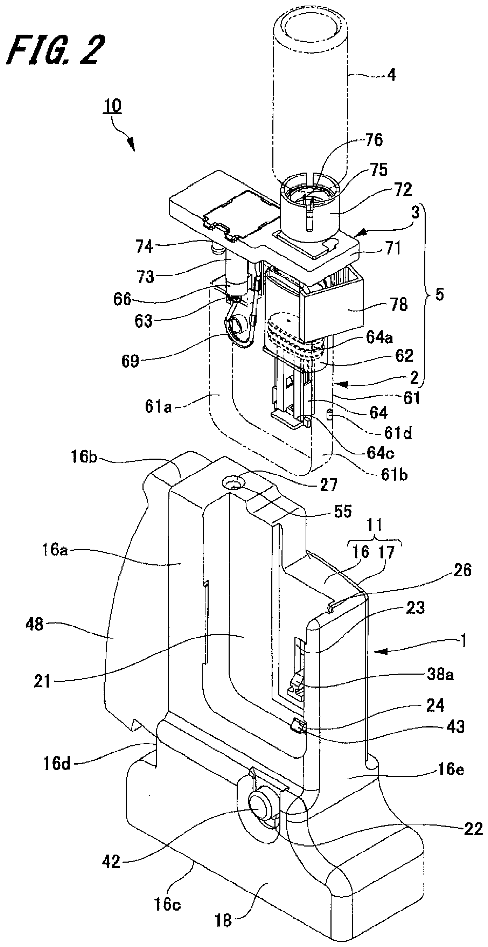

Fig. 1 is a perspective view of the drug solution filling device of the present example viewed from front, and Fig. 2 is an exploded perspective view illustrating the drug solution filling device, a filling adapter, and a drug solution reservoir instrument of the present example. In Figs. 1 and 2, an exterior case 61 of the drug solution reservoir instrument 2 which will be described later is transparently illustrated so that a principal part of the drug solution reservoir instrument 2 is illustrated.

As illustrated in Fig. 1, the drug solution filling device 1 is a device that fills a drug solution reservoir part 62 of the drug solution reservoir instrument 2 with a drug solution. To the drug solution filling device 1, the drug solution reservoir instrument 2 constituting the drug solution administration device and a filling adapter 3 are attached. The filling adapter 3 is mounted on the drug solution reservoir instrument 2 in advance. The drug solution reservoir instrument 2 and the filling adapter 3 constitute the drug solution filling unit 5. The drug solution filling device 1 and the drug solution filling unit 5 mounted on the drug solution filling device 1 constitute a drug solution filling set 10.

To the filling adapter 3, a vial bottle 4 that represents an example of a drug solution container is attached. The drug solution reservoir instrument 2 is integrally combined with a drive unit to form the drug solution administration device. The drive unit has, for example, a power source and a drive mechanism that supplies the drug solution. The drug solution administration device is widely applied to portable drug solution administration devices for continually administering a drug solution into a body of a patient. Examples of the portable drug solution administration devices include a patch insulin pump, a tube insulin pump, and other portable drug solution administration devices.

### [Drug Solution Filling Device]

Next, the drug solution filling device 1 will be described with reference to Figs. 1 to 6.

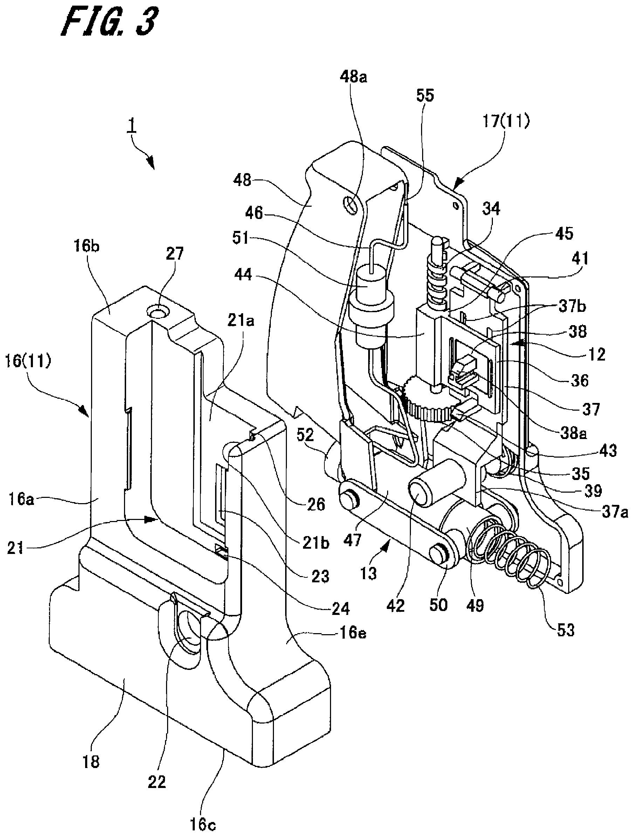

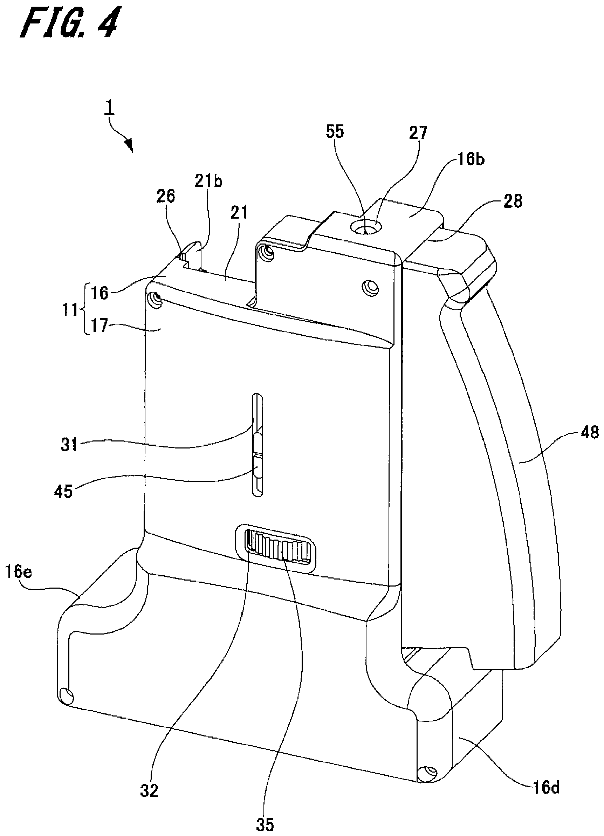

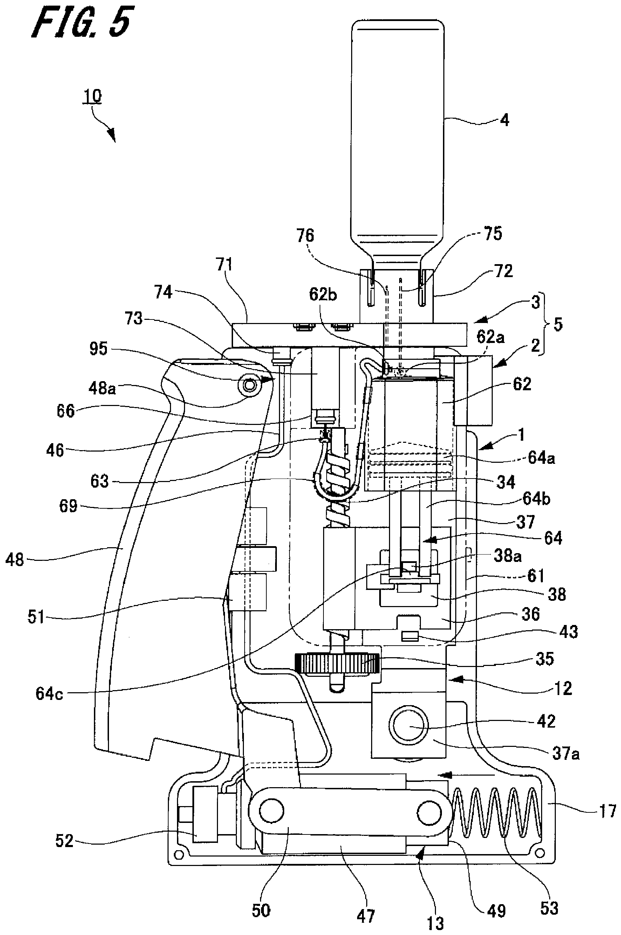

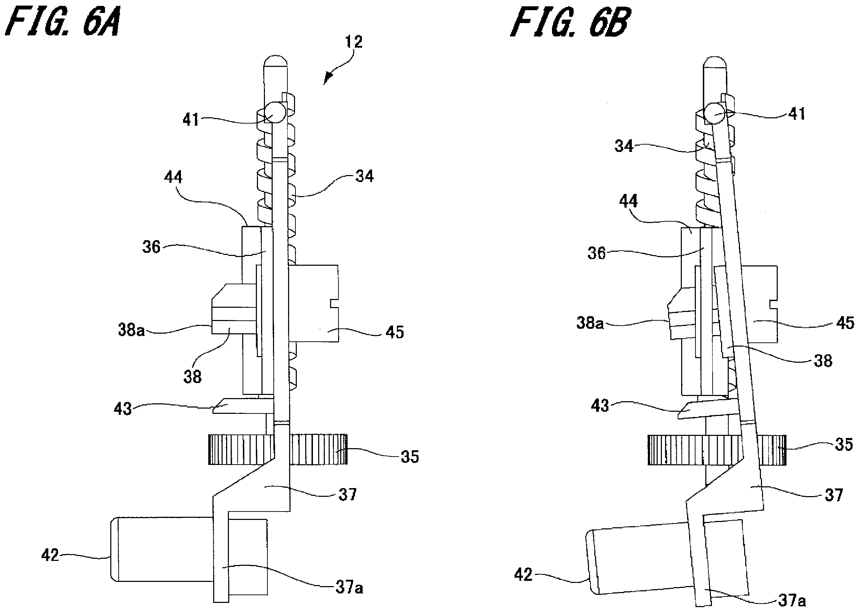

Fig. 3 is an exploded perspective view illustrating the drug solution filling device 1 of the present example. Fig. 4 is a perspective view of the drug solution filling device 1 of the present example viewed from back. Fig. 5 is a front view illustrating the drug solution filling set 10. In Fig. 5, the drug solution filling device 1 from which a first case 16 has been removed is illustrated. Figs. 6A and 6B are side views illustrating a filling amount adjustment mechanism 12 which will be described later.

As illustrated in Figs. 3 and 4, the drug solution filling device 1 has a housing 11 constituting an appearance configuration, the filling amount adjustment mechanism 12 provided in the housing 11, and a suction mechanism 13.

### [Housing]

The housing 11 is formed in a substantially rectangular solid shape, a portion of which is notched. The housing 11 is also formed so as to be able to be grasped by a user. The housing 11 has the first case 16 and a second case 17 that overlap each other.

The first case 16 has a front face part 16a, an upper face part 16b, and a placement face part 16c. The front face part 16a is formed in a substantially rectangular shape. The upper face part 16b substantially vertically continues from one end in a longitudinal direction of the front face part 16a. The placement face part 16c substantially vertically continues from the other end in the longitudinal direction of the front face part 16a and faces the upper face part 16b. The first case 16 also has a first side face part 16d and a second side face part 16e. The first side face part 16d substantially vertically continues from one end in a lateral direction of the front face part 16a. The second side face part 16e substantially vertically continues from the other side in the lateral direction of the front face part 16a and faces the first side face part 16d. The placement face part 16c is placed on a table or a board, whereby the drug solution filling device 1 is erected so that a longitudinal direction of the housing 11 is directed in a vertical direction.

On the other side in the longitudinal direction of the front face part 16a, a base part 18 protruding toward both sides in the lateral direction is provided. In the base part 18, an insertion hole 22 opened in a substantially circular shape is formed.

A mounting part 21 is provided above the base part 18 of the front face part 16a. The mounting part 21 is formed to range from the front face part 16a to the upper face part 16b. The mounting part 21 is recessed from one face of the front face part 16a in a width direction orthogonal to both the longitudinal direction and the lateral direction of the front face part 16a. As illustrated in Figs. 1 and 2, the drug solution filling unit 5 is mounted on the mounting part 21.

In a main face part 21a of the mounting part 21 in parallel with the one face of the front face part 16a, an opening part 23 opened in a substantially quadrilateral shape and a through hole 24 are formed. In a side wall 21b of the mounting part 21 on a side close to the second side face part 16e, a positioning groove 26 is provided.

On one side in the longitudinal direction of the front face part 16a, which is the other face on the opposite side of the exposed one face, a pivot pin, a first bearing part, and a second bearing part are provided (not illustrated). A lever member 48 of the suction mechanism 13 which will be described later is supported by the pivot pin so as to be capable of pivoting. A feed screw shaft 34 of the filling amount adjustment mechanism 12 which will be described later is supported by the first bearing part so as to be capable of rotating. A swing pin 41 of a guide member 37 of the filling amount adjustment mechanism 12 is supported by the second bearing part so as to be capable of pivoting.

A suction side connection hole 27 is provided in the upper face part 16b. A second connection port of the filling adapter 3 which will be described later is inserted into the suction side connection hole 27. A suction side connection port 55 of the suction mechanism 13 which will be described later is arranged in the suction side connection hole 27.

As illustrated in Fig. 4, a notch part 28 notched in a substantially quadrilateral shape is formed in the first side face part 16d. The lever member 48 of the suction mechanism 13 which will be described later is arranged in the notch part 28.

The second case 17 is formed in a flat plate shape having a substantially rectangular shape and arranged on a back side of the housing 11. The second case 17 covers an opening formed by the upper face part 16b, the first side face part 16d, the second side face part 16e, and the placement face part 16c of the first case 16. The other side in a longitudinal direction of the second case 17 protrudes toward both sides in a lateral direction in the same way as the front face part 16a of the first case 16.

As illustrated in Fig. 4, a long hole 31 and an operation window 32 are provided in the second case 17. The long hole 31 is opened substantially in the center of the second case 17 along the longitudinal direction by a predetermined length. In the vicinity of the long hole 31 of the second case 17, a filling amount adjustment scale is described. The filling amount adjustment scale may be indicated on one face of the second case 17 by printing, or may be molded integrally with the second case 17. The operation window 32 is arranged on the other side in the longitudinal direction of the second case beyond the long hole 31.

### [Filling Amount Adjustment Mechanism]

Next, a configuration of the filling amount adjustment mechanism 12 will be described.

As illustrated in Figs. 3 and 5, the filling amount adjustment mechanism 12 has the feed screw shaft 34, an operation part 35, a movable member 36, the guide member 37, and a hook member 38.

The feed screw shaft 34 is supported by the first bearing part (not illustrated) provided on the first case 16 so as to be capable of rotating. The feed screw shaft 34 is provided with a screw groove extending in a spiral shape in its outer peripheral face. The feed screw shaft 34 is configured such that its axial direction is arranged along the longitudinal direction of the housing 11. On the other side in the axial direction of the feed screw shaft 34, the operation part 35 is provided. The operation part 35 is formed in a substantially disk shape and fixed to the feed screw shaft 34. A portion of the operation part 35 is exposed from the operation window 32 of the second case 17. When a user rotates the operation part 35, the feed screw shaft 34 is rotated.

The guide member 37 is formed in a flat plate shape having a substantially rectangular shape. On one end in a longitudinal direction of the guide member 37, the swing pin 41 is provided. The swing pin 41 of the guide member 37 is attached to the second bearing part (not illustrated) provided on the first case 16 so as to be capable of pivoting. Consequently, as illustrated in Fig. 6B, the guide member 37 pivots about the swing pin 41.

On the other side in the longitudinal direction of the guide member 37, a bending piece 37a is provided. A releasing button 42 projects from one face of the bending piece 37a. As illustrated in Fig. 2, the releasing button 42 is inserted into the insertion hole 22 of the first case 16. As illustrated in Fig. 3, a return spring 39 is arranged between the bending piece 37a and the second case 17.

Between the swing pin 41 and the bending piece 37a of the guide member 37, two guide grooves 37b are formed. The two guide grooves 37b are formed along the longitudinal direction of the guide member 37. Between the two guide grooves 37b and the bending piece 37a of the guide member 37, an engaging piece 43 is formed. As illustrated in Fig. 6A, the engaging piece 43 projects in the same direction as the releasing button 42. As illustrated in Fig. 2, the engaging piece 43 passes through the through hole 24 of the first case 16.

The movable member 36 is formed in a substantially flat plate shape, and an opening part 36a opened in a substantially quadrilateral shape is formed substantially in the center of the movable member 36. On one side of the movable member 36, a nut part 44 is provided. The nut part 44 is engaged with the feed screw shaft 34. Between the movable member 36 and the nut part 44, an indication piece 45 is provided. The indication piece 45 projects from the movable member 36 toward the second case 17. The indication piece 45 is inserted into the long hole 31 provided in the second case 17. A user can check a position of the movable member 36 by visually checking a position of the indication piece 45.

The hook member 38 is inserted into the opening part 36a of the movable member 36. The hook member 38 has a sliding piece (not illustrated) and a hook piece 38a. The sliding piece is inserted into the guide grooves 37b of the guide member 37 so as to be capable of sliding. As illustrated in Fig. 6A, the hook piece 38a projects in the same direction as the engaging piece 43. The hook piece 38a is engaged with a nut piece 64c of the drug solution reservoir instrument 2 which will be described later.

When the feed screw shaft 34 is rotated, the rotating force of the feed screw shaft 34 is transmitted to the movable member 36 and the hook member 38 through the nut part 44. The movable member 36 and the hook member 38 then move along the guide grooves 37b of the guide member 37. As a result, a position of the hook piece 38a can be adjusted.

The hook member 38 is attached to the guide member 37 through the sliding piece. As illustrated in Fig. 6B, therefore, when the guide member 37 pivots, the hook member 38 moves together with the guide member 37.

### [Suction Mechanism]

Next, a configuration of the suction mechanism 13 will be described.

As illustrated in Figs. 3 and 5, the suction mechanism 13 has the suction side connection port 55, a suction tube 46, a syringe 47, the lever member 48, a suction side pusher 49, and a link member 50 that connects the lever member 48 to the suction side pusher 49.

As illustrated in Fig. 2, the suction side connection port 55 is arranged in the suction side connection hole 27 provided in the housing 11. As illustrated in Figs. 3 and 5, the suction tube 46 that represents an example of a suction channel is connected to the suction side connection port 55. One end of the suction tube 46 is connected to the suction side connection port 55, and the other end of the suction tube 46 is connected to the syringe 47.

In the middle of the suction tube 46, a first check valve 51 is provided. The first check valve 51 is opened only when gas that passes through the suction tube 46 goes from the suction side connection port 55 to the syringe 47 and closed when gas goes from the syringe 47 to the suction side connection port 55.

The syringe 47 is formed in a substantially cylindrical shape. On one end part in an axial direction of the syringe 47, a second check valve 52 is provided. The second check valve 52 is opened only when gas is discharged from the syringe 47 and closed when gas is sucked in the syringe 47 from the outside other than the suction tube 46. Therefore, only gas that passes through the suction tube 46 is sent into the syringe 47.

The suction side pusher 49 is invaginated into a cylinder hole of the syringe 47 in the axial direction of the syringe 47 so as to be capable of sliding. The suction side pusher 49 freely adjusts the volume in the syringe 47. The suction side pusher 49 is provided with a pressing spring 53. The pressing spring 53 is interposed between the suction side pusher 49 and the second side face part 16e of the first case 16. The pressing spring 53 energizes the suction side pusher 49 to one side in the axial direction of the syringe 47.

The lever member 48 is coupled to the suction side pusher 49 through the link member 50. In one end part of the lever member 48, a bearing hole 48a is provided. The pivot pin (not illustrated) provided on the first case 16 passes through the bearing hole 48a. The lever member 48 is supported by the first case 16 and the second case 17 so as to be capable of pivoting about the pivot pin.

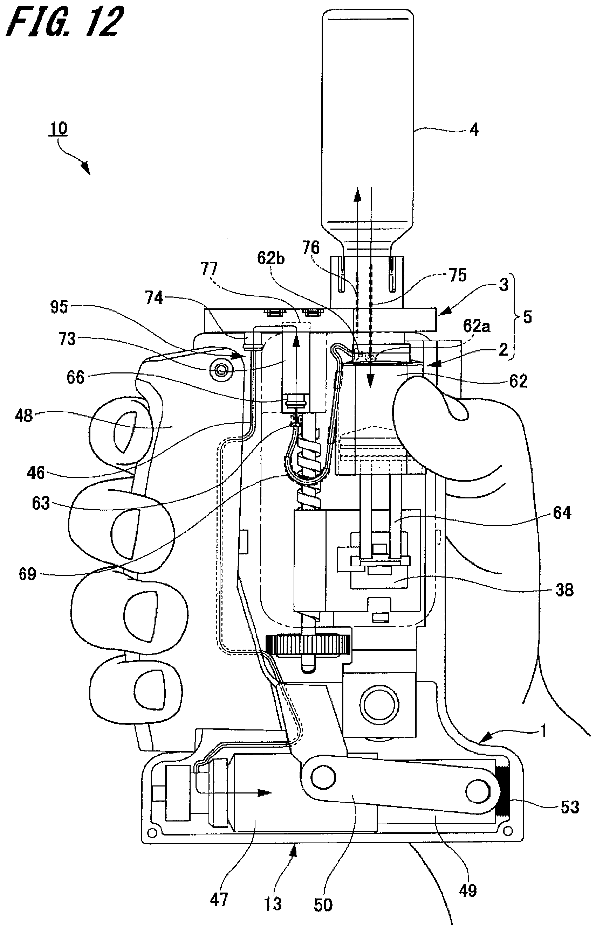

To the other end part of the lever member 48, one end part of the link member 50 is attached so as to be capable of pivoting. The other end part of the link member 50 is attached to the suction side pusher 49 so as to be capable of pivoting. When a user grips the lever member 48 and causes the lever member 48 to pivot about the pivot pin, the suction side pusher 49, together with the link member 50, moves to the other side in the axial direction of the syringe 47 against the energizing force of the pressing spring 53 (refer to Fig. 12). Consequently, the volume in the syringe 47 is increased, and air is sent into the syringe 47 through the suction side connection port 55 and the suction tube 46.

When the user stops the grip operation for the lever member 48, the suction side pusher 49 is energized by the pressing spring 53 and inserted into the syringe 47. The suction tube 46 is provided with the first check valve 51 that shuts off air that goes from the syringe 47 to the suction side connection port 55. Air within the syringe 47, therefore, is discharged to the outside through the second check valve 52. The moving force of the suction side pusher 49 is transmitted to the lever member 48 through the link member 50, and the lever member 48 returns to the initial state illustrated in Fig. 5.

### [Drug Solution Filling Unit]

Next, the drug solution filling unit 5 including the drug solution reservoir instrument 2 and the filling adapter 3 will be described with reference to Figs. 1, 2, 5, and 7 to 11.

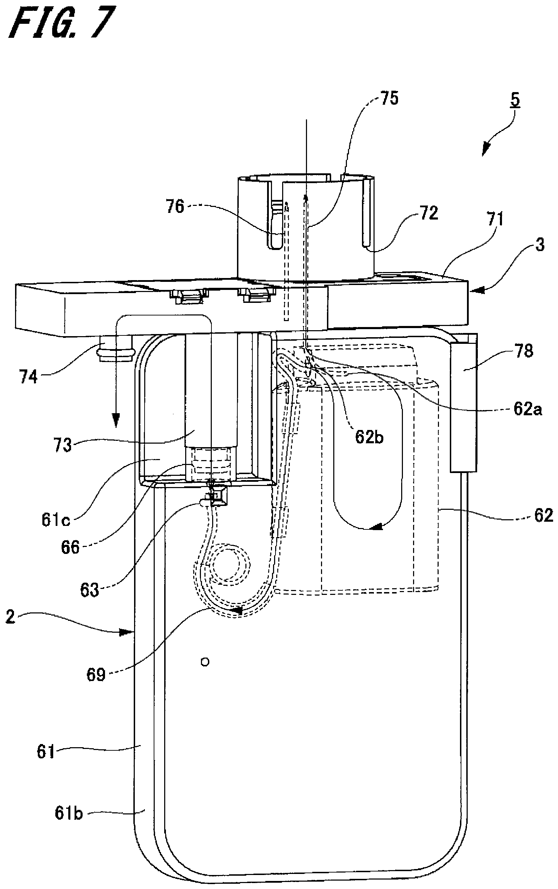

Fig. 7 is a perspective view illustrating the drug solution filling unit 5.

### [Drug Solution Reservoir Instrument]

As illustrated in Figs. 2 and 7, the drug solution reservoir instrument 2 has the exterior case 61, the drug solution reservoir part 62, a reservoir part side connection port 63, and a pusher member 64. One face of the exterior case 61 is opened. The drug solution reservoir part 62 is housed inside the exterior case 61.

The exterior case 61 is formed in a container shape, one face of which is opened. The exterior case 61 has a main face part 61a, a side wall 61b, and a recessed part 61c. The main face part 61a is formed in a substantially rectangular shape and surrounded by the side wall 61b. The recessed part 61c is recessed by one stage from the main face part 61a. The recessed part 61c is provided with a reservoir part side connection cylinder 66 formed in a substantially cylindrical shape. The side wall 61b of the exterior case 61 is provided with a positioning projection 61d (refer to Fig. 2). The positioning projection 61d is inserted into the positioning groove 26 provided in the mounting part 21 when the drug solution filling unit 5 is attached to the drug solution filling device 1.

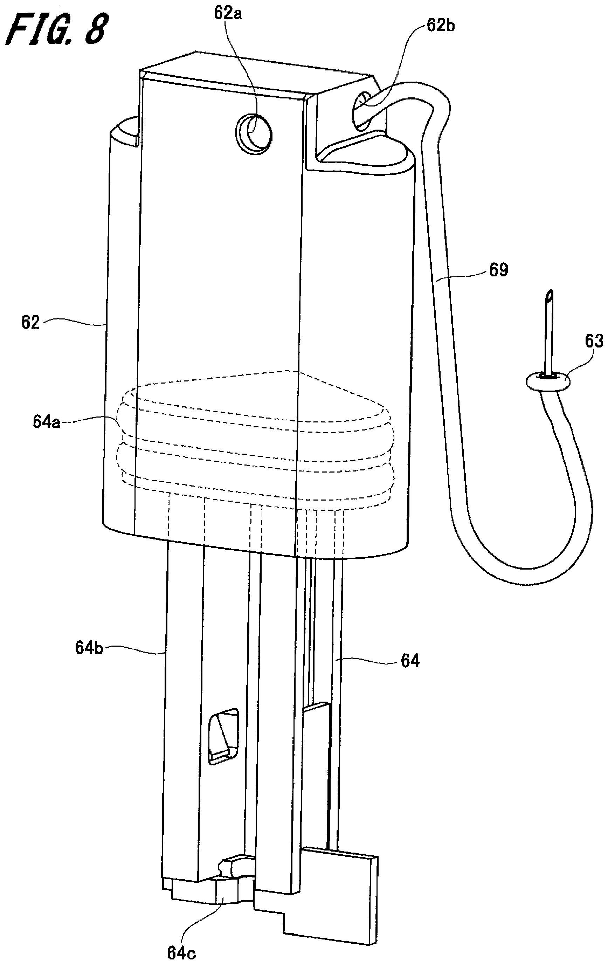

Fig. 8 is a perspective view illustrating a principal part in the drug solution reservoir instrument 2.

As illustrated in Fig. 8, the drug solution reservoir part 62 is formed in a substantially tubular shape. One face of the drug solution reservoir part 62 is closed, and the other face is opened. In an end part on the closed side of the drug solution reservoir part 62, a filling port 62a and a liquid supply port 62b are provided. The drug solution is injected from the filling port 62a, and air or the drug solution is sent from the liquid supply port 62b. As illustrated in Fig. 5, when the drug solution filling unit 5 is mounted on the drug solution filling device 1, the liquid supply port 62b is arranged vertically above the filling port 62a.

As illustrated in Fig. 7, a liquid supply tube 69 is connected to the liquid supply port 62b. The reservoir part side connection port 63 is connected to an end part of the liquid supply tube 69 on the opposite side of the liquid supply port 62b. The reservoir part side connection port 63 is arranged in the reservoir part side connection cylinder 66 of the exterior case 61.

The pusher member 64 is inserted into a cylinder hole of the drug solution reservoir part 62 so as to be capable of sliding. The pusher member 64 has a gasket 64a, a shaft part 64b, and the nut piece 64c. The gasket 64a moves in close contact with an inner peripheral face of the drug solution reservoir part 62 in a liquid-tight manner. The shaft part 64b is attached to the gasket 64a. The nut piece 64c is fixed to the shaft part 64b. The nut piece 64c is provided on the shaft part 64b on the opposite side of the gasket 64a. When the drug solution reservoir instrument 2 and the drive unit are integrally combined, the nut piece 64c is engaged with a screw shaft of the drive mechanism provided at the drive unit.

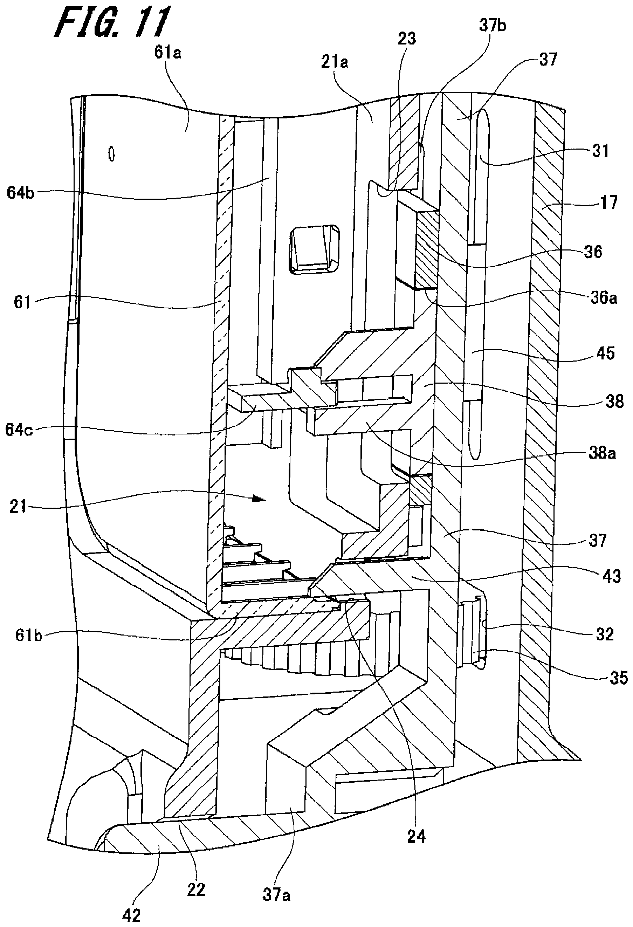

When the drug solution filling unit 5 is attached to the drug solution filling device 1, the nut piece 64c is engaged with the hook piece 38a of the filling amount adjustment mechanism 12 (refer to Fig. 11).

### [Filling Adapter]

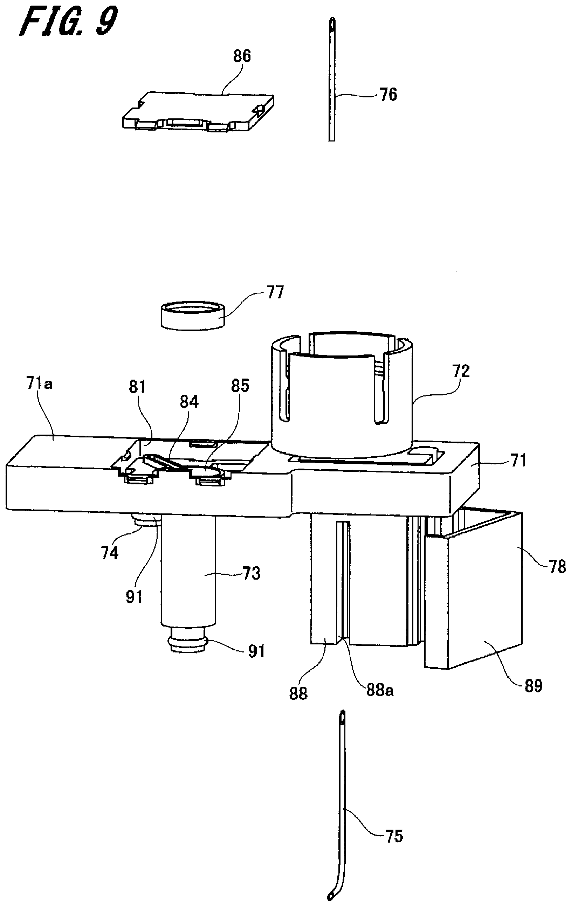

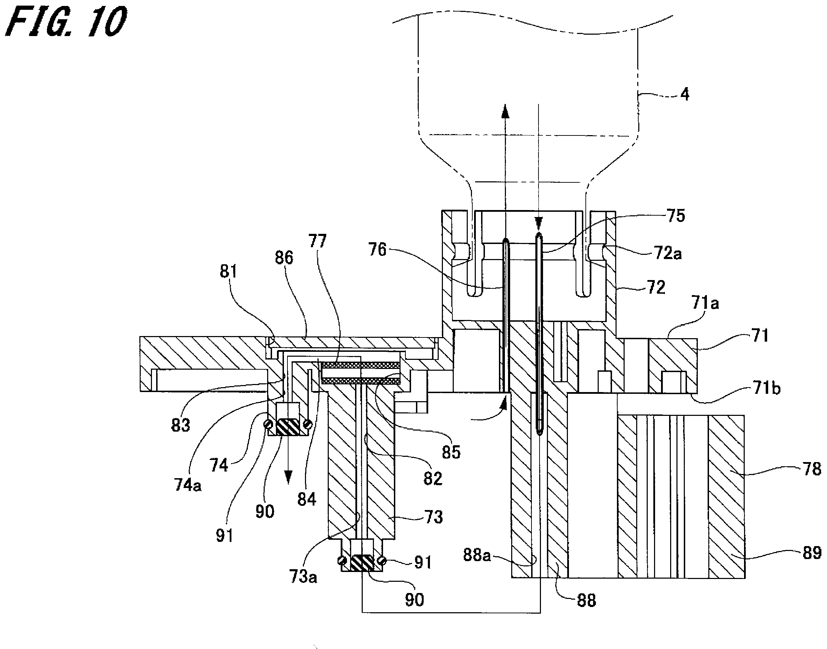

Next, the filling adapter 3 will be described with reference to Figs. 9 and 10.

Fig. 9 is an exploded perspective view illustrating the filling adapter 3, and Fig. 10 is a cross-sectional view illustrating the filling adapter 3.

As illustrated in Figs. 9 and 10, the filling adapter 3 includes a main body part 71, a supporting part 72, a first connection port 73, the second connection port 74, a liquid supply needle tube 75, an air vent needle tube 76, and an air vent filter 77. The liquid supply needle tube 75 and the air vent needle tube 76 are held by the main body part 71. The filling adapter 3 also has a protection mechanism 78 provided on the main body part 71.

### [Main Body Part]

The main body part 71 is formed in a substantially rectangular solid shape. On one side in a longitudinal direction of the main body part 71, the supporting part 72, the protection mechanism 78, the liquid supply needle tube 75, and the air vent needle tube 76 are arranged. On the other side in the longitudinal direction of the main body part 71, the first connection port 73 and the second connection port 74 are arranged.

On the other side in the longitudinal direction of the main body part 71, a recess 81 recessed in a substantially quadrilateral shape from one face 71a is formed. The recess 81 is provided at a position corresponding to those of the first connection port 73 and the second connection port 74 provided on the main body part 71. In the recess 81, a first communication hole 82 and a second communication hole 83 are formed. The first communication hole 82 and the second communication hole 83 each penetrate the main body part 71 in an up-and-down direction. A communication passage 84 that causes the first communication hole 82 and the second communication hole 83 to communicate with each other is formed in the recess 81.

An outer edge of the first communication hole 82 on a side close to the recess 81 is provided with a mounting hole 85. The air vent filter 77 is mounted in the mounting hole 85. The air vent filter 77 has such a mechanism as to allow only gas such as air to pass through and not allow liquid to pass through. The main body part 71 is provided with a lid part 86 that closes an opening of the recess 81.

### [Liquid Supply Needle Tube and Air Vent Needle Tube]

At one end of each of the liquid supply needle tube 75 and the air vent needle tube 76, a needle tip is provided. The one end of each of the liquid supply needle tube 75 and the air vent needle tube 76 punctures a rubber plug that seals an opening of the vial bottle 4 and penetrates the rubber plug. The other end of the liquid supply needle tube 75 is also provided with a needle tip. The other end of the liquid supply needle tube 75 punctures the filling port 62a of the drug solution reservoir part 62 and penetrates the filling port 62a. The other end side of the liquid supply needle tube 75 is bent substantially in an L shape.

The liquid supply needle tube 75 and the air vent needle tube 76 are held by the main body part 71. The one end side of each of the liquid supply needle tube 75 and the air vent needle tube 76 projects from the one face 71a of the main body part 71. The other end side of the liquid supply needle tube 75 projects from the other face 71b that is the opposite face of the one face 71a of the main body part 71. In other words, the liquid supply needle tube 75 is provided to penetrate the main body part 71 in the up-and-down direction. The other end of the air vent needle tube 76 projects from the other face 71b of the main body part 71. Alternatively, an end face of the other end of the air vent needle tube 76 is located on the same plane as the other face 71b. The other end of the air vent needle tube 76 may not project from the other face 71b of the main body part 71. For example, an air vent hole communicating with the air vent needle tube 76 may be provided in the main body part 71.

On the one side in the longitudinal direction of the main body part 71, the supporting part 72 formed in a substantially cylindrical shape is provided. An opening of the supporting part 72 is configured to have such a size that a mouth part of the vial bottle 4 can be inserted thereinto. In a cylinder hole of the supporting part 72, the one end side of each of the liquid supply needle tube 75 and the air vent needle tube 76 is arranged. Specifically, the supporting part 72 is formed in a tubular shape that covers peripheries of the liquid supply needle tube 75 and the air vent needle tube 76, and provided apart in a radial direction from the needle tip at the one side of each of the liquid supply needle tube 75 and the air vent needle tube 76. Consequently, a user can be prevented from being punctured by mistake by the needle tip at the one end side of each of the liquid supply needle tube 75 and the air vent needle tube 76.

In an inner wall of the supporting part 72, a rib 72a continuing in a circumferential direction is formed. The rib 72a is fit with the mouth part of the vial bottle 4. As a result, the vial bottle 4 can be held by the supporting part 72.

The protection mechanism 78 is provided on the other face 71b of the main body part 71 and arranged in the vicinity of the other end of the liquid supply needle tube 75 of the main body part 71. The protection mechanism 78 has a first cover part 88 and a second cover part 89. The first cover part 88 projects from the other face 71b of the main body part 71.

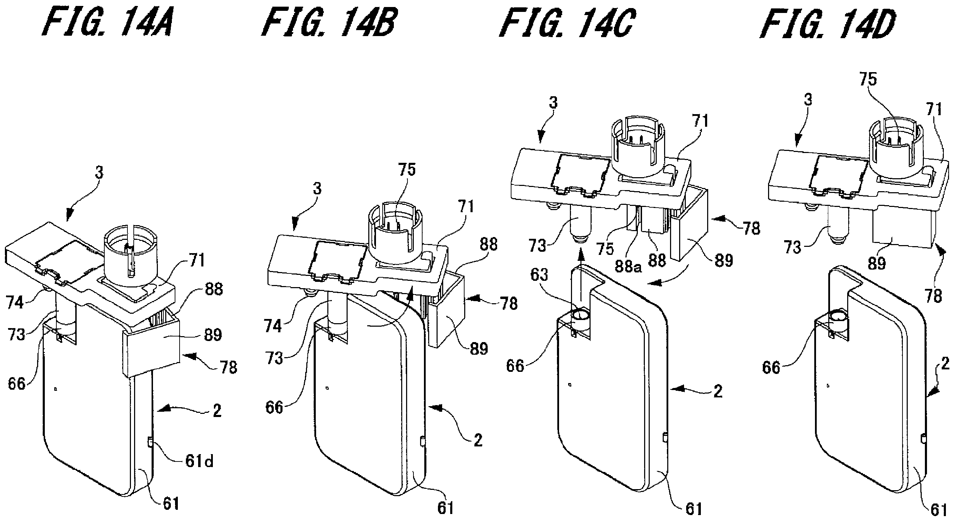

The first cover part 88 that represents an example of a protection cover is formed in a substantially flat plate shape. A groove part 88a is formed in the first cover part 88. The other end side of the liquid supply needle tube 75 held by the main body part 71 is inserted into the groove part 88a. The second cover part 89 that represents an example of a protection cover is attached to the first cover part 88 so as to be capable of pivoting. The second cover part 89 pivots and thus covers the groove part 88a of the first cover part 88 and the other end side of the liquid supply needle tube 75 (refer to Fig. 14D). Consequently, the needle tip at the other end side of the liquid supply needle tube 75 can be covered after filling work is finished, and the used filling adapter 3 can be kept in a safe state.

In the present example, an example where the first cover part 88 and the second cover part 89 are used as a configuration of the protection mechanism 78 has been described. However the configuration of the protection mechanism 78 is not limited to this example. For example, a portion of the main body part that supports the liquid supply needle tube 75 may be configured to be capable of pivoting, and the needle tip at the other end side of the liquid supply needle tube 75 may be covered with a protection cover provided on the main body part when the portion is caused to pivot.

The first connection port 73 and the second connection port 74 are provided on the other face 71b of the main body part 71 on a side close to the recess 81. The first connection port 73 and the second connection port 74 are each formed in a substantially columnar shape. Communication holes 73a, 74a penetrating along axial directions are respectively formed in the first connection port 73 and the second connection port 74. Sealing bodies 90, 90 are attached to opening parts of the communication holes 73a, 74a on the opposite side of the main body part 71 so as to close the openings.

The first connection port 73 communicates with the first communication hole 82 provided in the main body part 71, and the second connection port 74 communicates with the second communication hole 83. A seal member 91 is provided on an end part of each of the first connection port 73 and the second connection port 74 on the opposite side of the main body part 71. As illustrated in Fig. 7, the first connection port 73 is inserted into the reservoir part side connection cylinder 66 of the drug solution reservoir instrument 2 and connected to the reservoir part side connection port 63. The second connection port 74 is inserted into the suction side connection hole 27 of the drug solution filling device 1 and connected to the suction side connection port 55.

### 1-2. Drug Solution Filling Method for Drug Solution Filling Set

Next, an example of a drug solution filling method that uses the drug solution filling set 10 of the present example will be described with reference to Figs. 1 to 13.

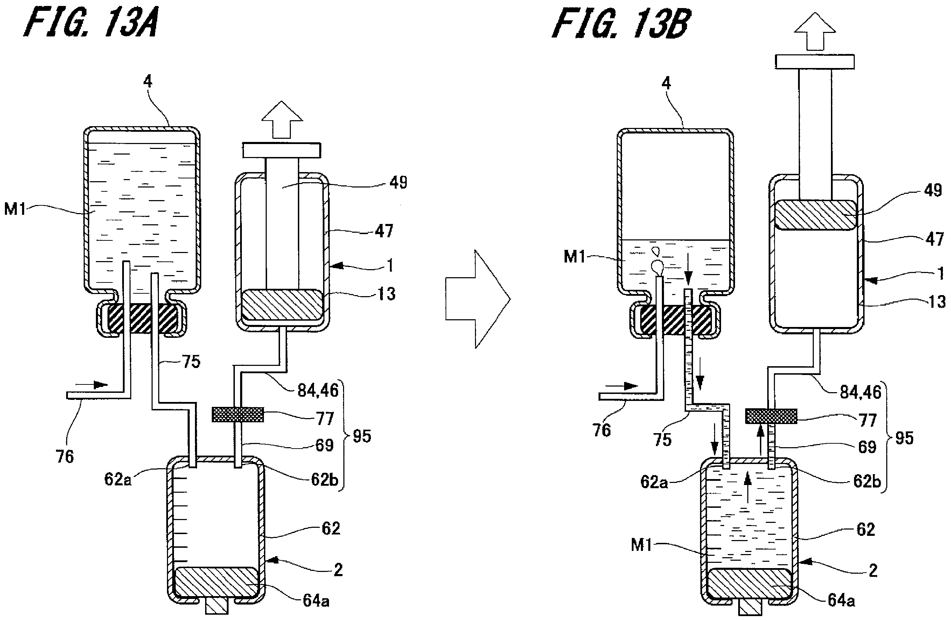

Fig. 11 is a cross-sectional view illustrating a principal part in the drug solution filling set 10, and Fig. 12 is a front view of the drug solution filling set 10 illustrating operation for filling the drug solution reservoir part 62 of the drug solution reservoir instrument 2 with the drug solution. Figs. 13A and 13B are explanatory views schematically illustrating the drug solution filling operation.

First, as illustrated in Fig. 2, a user erects the drug solution filling device 1 on a table or a board so that an opening of the mounting part 21 on a side close to the upper face part 16b is directed upward. On a lower part of the housing 11, the drug solution filling device 1 is provided with the base part 18 protruding toward both sides of the first side face part 16d and the second side face part 16e. As a result, the drug solution filling device 1 can stably stand on a table or a board.

Next, as illustrated in Fig. 4, the operation part 35 of the drug solution filling device 1 is operated, and the indication piece 45 is set to a predetermined position on the scale. Consequently, as illustrated in Figs. 2 and 3, the movable member 36 and the hook member 38 move to a predetermined position. The setting for the hook member 38 of the filling amount adjustment mechanism 12 may be performed before the drug solution filling device 1 is placed on a table or a board, or may be performed after the drug solution filling unit 5 is mounted on the drug solution filling device 1.

The filling adapter 3 is mounted on the drug solution reservoir instrument 2 in advance. The other end side of the liquid supply needle tube 75 of the filling adapter 3 penetrates the filling port 62a of the drug solution reservoir part 62. The first connection port 73 is inserted into the reservoir part side connection cylinder 66 of the drug solution reservoir instrument 2, and the reservoir part side connection port 63 penetrates the sealing body 90 of the first connection port 73. As a result, the liquid supply port 62b, the liquid supply tube 69, and the reservoir part side connection port 63 of the drug solution reservoir instrument 2 communicate with the first connection port 73 of the filling adapter 3.

Next, as illustrated in Figs. 1 and 5, the drug solution filling unit 5 is mounted on the drug solution filling device 1. Specifically, the projection 61d of the drug solution reservoir instrument 2 is inserted into the positioning groove 26 of the mounting part 21, and the drug solution reservoir instrument 2 is mounted on the mounting part 21. The second connection port 74 of the filling adapter 3 is inserted into the suction side connection hole 27 of the drug solution filling device 1. The seal member 91 (refer to Fig. 10) of the second connection port 74 then comes into close contact with the suction side connection hole 27. The suction side connection port 55 penetrates the sealing body 90 of the second connection port 74. As a result, the second connection port 74 and the suction side connection port 55 communicate with each other. Then, as illustrated in Fig. 13A, an internal space of the syringe 47 of the suction mechanism 13 and an internal space of the drug solution reservoir part 62 communicate with each other through the liquid supply port 62b, the liquid supply tube 69, the communication passage 84, and the suction tube 46. The liquid supply tube 69, the communication passage 84, and the suction tube 46 constitute a suction channel 95.

As illustrated in Fig. 11, the engaging piece 43 projecting from the mounting part 21 is engaged with the side wall 61b of the exterior case 61 of the drug solution reservoir instrument 2. Consequently, the drug solution reservoir instrument 2 can be stably mounted on the mounting part 21.

The hook piece 38a of the filling amount adjustment mechanism 12 is engaged with the nut piece 64c of the drug solution reservoir instrument 2, and an insertion position of the pusher member 64 with respect to the drug solution reservoir part 62 is maintained at a predetermined position. As a result, as illustrated in Fig. 5, a position of the gasket 64a of the pusher member 64 in the drug solution reservoir part 62 is maintained, and the volume in the drug solution reservoir part 62 can be maintained at a predetermined amount. Even after the drug solution reservoir instrument 2 is mounted on the mounting part 21, the operation part 35 can be operated to change the position of the hook piece 38a engaged with the nut piece 64c, whereby the volume of the drug solution reservoir part 62 can be easily changed.

Next, the vial bottle 4 containing the drug solution is attached to the supporting part 72 of the filling adapter 3. Consequently, the one end side of each of the liquid supply needle tube 75 and the air vent needle tube 76 of the filling adapter 3 penetrates the rubber plug of the vial bottle 4, and the liquid supply needle tube 75 and the air vent needle tube 76 communicate with an internal space of the vial bottle 4. The other end of the liquid supply needle tube 75 penetrates the filling port 62a of the drug solution reservoir part 62. As illustrated in Fig. 13A, therefore, the internal space of the vial bottle 4 and the internal space of the drug solution reservoir part 62 communicate with each other through the liquid supply needle tube 75.

The vial bottle 4 may be attached to the filling adapter 3 before the drug solution filling unit 5 is mounted on the drug solution filling device 1.

Next, as illustrated in Fig. 12, the user grasps the housing 11 and grips the lever member 48 to cause the lever member 48 to pivot. When the lever member 48 pivots, the suction side pusher 49 moves through the link member 50, and the volume in the syringe 47 is increased. The syringe 47 is provided with the second check valve 52 that is opened only when gas is discharged from the syringe 47 and closed when gas is sucked in the syringe 47. The syringe 47, therefore, sucks in air from a side close to the suction tube 46.

Consequently, as illustrated in Figs. 12 and 13A, air within the drug solution reservoir part 62 is sucked in the syringe 47 through the suction channel 95. As a result, the drug solution reservoir part 62 has a negative pressure inside.

Since the drug solution reservoir part 62 has a negative pressure inside, the drug solution M1 is injected from the vial bottle 4 into the drug solution reservoir part 62 through the liquid supply needle tube 75 and the filling port 62a. External air is sent into the vial bottle 4 through the air vent needle tube 76. In this manner, the drug solution M1 can be easily injected into the drug solution reservoir part 62 when the user grips the lever member 48.

When the user stops the grip operation for the lever member 48, the suction side pusher 49 is energized by the pressing spring 53 and inserted into the syringe 47. The suction tube 46 is provided with the first check valve 51 that shuts off air that goes from the syringe 47 to the suction side connection port 55. Furthermore, as described above, the syringe 47 is provided with the second check valve 52 that is opened only when gas is discharged from the syringe 47 and closed when gas is sucked in the syringe 47. Air within the syringe 47, therefore, is discharged to the outside through the second check valve 52, not through a side close to the suction tube 46. The moving force of the suction side pusher 49 is transmitted to the lever member 48 through the link member 50, and the lever member 48 returns to the initial state illustrated in Fig. 5.

The user then repeats the grip operation for the lever member 48. As a result, air within the drug solution reservoir part 62 is sucked by the suction mechanism 13 of the drug solution filling device 1, and the drug solution reservoir part 62 has a negative pressure inside, whereby the drug solution M1 is injected from the vial bottle 4 into the drug solution reservoir part 62.

When the drug solution reservoir part 62 is occupied by the drug solution M1, as illustrated in Fig. 13B, the liquid supply tube 69 is also filled with the drug solution M1. The suction channel 95 is provided with the air vent filter 77 that allows only gas such as air to pass through and does not allow liquid to pass through. When the drug solution reservoir part 62 and the suction channel 95 are occupied by the drug solution M1 up to the air vent filter 77, and air disappears, the suction mechanism 13 can no longer suck in air, and the pivot operation for the lever member 48 becomes stiff. In this manner, the user can be notified that the drug solution reservoir part 62 has been filled with the drug solution M1.

The liquid supply port 62b of the drug solution reservoir part 62 from which air is sucked is arranged vertically above the filling port 62a from which the drug solution M1 is injected. Furthermore, the suction mechanism 13 of the drug solution filling device 1 sucks in air within the drug solution reservoir part 62, whereby air within the drug solution reservoir part 62 can be completely discharged from the liquid supply port 62b. As a result, it is possible to omit work of operating the pusher member 64 provided in the drug solution reservoir instrument 2 after the drug solution filling work to remove remaining air from the drug solution reservoir part 62. Since the pusher member 64 does not need to be operated, the volume of the drug solution reservoir part 62 never changes, and the drug solution reservoir part 62 can be filled with an appropriate amount. It is also possible to eliminate discharge of the drug solution M1 that occurs at the time of the air removal.

Furthermore, the filling work can be performed while the liquid supply port 62b and the filling port 62a are arranged vertically above the gasket 64a of the pusher member 64 by the drug solution filling device 1.

Next, the drug solution filling unit 5 is removed from the drug solution filling device 1. Specifically, the releasing button 42 is pressed. Consequently, as illustrated in Fig. 6B, the guide member 37 pivots about the swing pin 41. As a result, the engagement between the engaging piece 43 and the exterior case 61 is released, and the engagement between the hook piece 38a and the nut piece 64c is also released.

Next, the drug solution filling unit 5 is pulled out upward. Consequently, the second connection port 74 is pulled out of the suction side connection hole 27, and the drug solution reservoir part 2 is removed from the mounting part 21. The drug solution filling work for the drug solution reservoir part 62 is thus completed. The vial bottle 4 may be removed from the filling adapter 3 before the drug solution filling unit 5 is removed from the drug solution filling device 1.

### 1-3. Method for Removing Filling Adapter 3

Next, a method for removing the filling adapter 3 from the drug solution reservoir instrument 2 of the present example will be described with reference to Figs. 14A to 14D.

Figs. 14A to 14D are explanatory views illustrating the method for removing the filling adapter 3.

First, as illustrated in Fig. 14A, the drug solution filling unit 5 is removed from the drug solution filling device 1. Next, as illustrated in Fig. 14B, the filling adapter 3 is caused to pivot about the first connection port 73 in such a direction that a side close to the protection mechanism 78 of the filling adapter 3 is separated from the drug solution reservoir instrument 2. Consequently, the liquid supply needle tube 75 comes out of the filling port 62a (refer to Fig. 8) of the drug solution reservoir part 62.

Next, as illustrated in Fig. 14C, the first connection port 73 is pulled out of the reservoir part side connection cylinder 66, and the filling adapter 3 is removed from the drug solution reservoir instrument 2. Next, as illustrated in Fig. 14D, the second cover part 89 is caused to pivot in a direction approaching the first cover part 88. The needle tip at the other end side of the liquid supply needle tube 75 is then covered with the first cover part 88 and the second cover part 89. As a result, the needle tip of the used liquid supply needle tube 75 can be prevented from puncturing the user by mistake, and safety of the filling adapter 3 can be enhanced.

The work of removing the filling adapter 3 from the drug solution reservoir instrument 2 is thus completed.

The present invention is not limited to the embodiment described above and illustrated in the drawings, and can be variously changed and implemented in a range not departing from the gist of the invention described in the claims. For example, although an example where the syringe 47, the suction side pusher 49, and the link member 50 are used as the suction mechanism 13 has been described in the above-mentioned embodiment, the present invention is not limited to this example. For example, an electric pump may be used as the suction mechanism, and other various suction mechanisms may also be applied.

The mounting part may be configured to cover the entire exterior case 61 of the drug solution reservoir instrument 2. In this case, the mounting part is preferably provided with a visual check window through which a state of the drug solution reservoir part 62 can be visually checked.

### Reference Signs List

- 1: drug solution filling device
- 2: drug solution reservoir instrument
- 3: filling adapter
- 4: vial bottle (drug solution container)
- 5: drug solution filling unit
- 10: drug solution filling set
- 11: housing
- 12: filling amount adjustment mechanism
- 13: suction mechanism
- 16: first case
- 17: second case
- 18: base part
- 21: mounting part
- 23: opening part
- 26: positioning groove
- 27: suction side connection hole
- 34: feed screw shaft
- 35: operation part
- 36: movable member
- 37: guide member
- 38: hook member
- 44: nut part
- 49: suction side pusher
- 50: link member
- 51: first check valve
- 52: second check valve
- 55: suction side connection port
- 61: exterior case
- 62: drug solution reservoir part
- 64c: nut piece
- 73: first connection port
- 74: second connection port
- 75: liquid supply needle tube
- 76: air vent needle tube
- 77: air vent filter
- 78: protection mechanism
- 95: suction channel
- M1: drug solution

## Claims

1. A drug solution filling unit (5) comprising:
a drug solution reservoir instrument (2) having a drug solution reservoir part (62) that is configured to reserve a drug solution (M1); and
a filling adapter (3) configured to be detachably mounted on the drug solution reservoir instrument (2), wherein
the drug solution reservoir instrument (2) includes:
the drug solution reservoir part (62) having a filling port (62a) from which the drug solution (M1) can be injected; and
an exterior case (61) that is configured to house the drug solution reservoir part (62), and
the filling adapter (3) includes:
a liquid supply needle tube (75) configured to be connected to the drug solution container (4) and the filling port (62a) and through which the drug solution (M1) that is sent from the drug solution container (4) to the drug solution reservoir part (62) can pass; **characterized by**
the drug solution reservoir part (62) further having a liquid supply port (62b) that is configured to send the filled drug solution (M1); and
the filling adapter (3) further including:
an air vent needle tube (76) configured to be connected to a drug solution container (4) and through which gas that is sent to the drug solution container (4) can pass; and
a suction channel (95) configured to communicate with the liquid supply port (62b) and configured to communicate with a suction mechanism (13) that is configured to suck in gas within the drug solution reservoir part (62).

2. The drug solution filling unit (5) according to claim 1, wherein
the suction channel (95) is provided with an air vent filter (77) that is configured to allow only gas to pass through and is configured to not allow the drug solution (M1) to pass through.

3. The drug solution filling unit (5) according to claim 1 or 2, wherein
the liquid supply port (62b) is arranged vertically above the filling port (62a) when the drug solution (M1) is filled.

4. The drug solution filling unit (5) according to any of claims 1 to 3, wherein
the filling adapter (3) has a protection mechanism (78) that is configured to cover a needle tip of the liquid supply needle tube (75) at a side configured to be connected to the liquid supply port (62b) after filling work for the drug solution (M1) is finished.

5. The drug solution filling unit (5) according to any of claims 1 to 4, wherein
the filling adapter (3) has a supporting part (72) that is configured to cover a needle tip of each of the air vent needle tube (76) and the liquid supply needle tube (75) at a side configured to be connected to the drug solution container (4), and is configured to support the drug solution container (4).

6. A drug solution reservoir instrument (2) comprising:
a drug solution reservoir part (62) having a filling port (62a) from which a drug solution (M1) can be injected and a liquid supply port (62b) that is configured to send the filled drug solution (M1); and
an exterior case (61) that is configured to house the drug solution reservoir part (62), wherein
gas within the drug solution reservoir part (62) is sucked from the liquid supply port (62b) when the drug solution (M1) is filled.

7. A filling adapter (3) comprising:
a main body part (71) configured to be detachably mounted on a drug solution reservoir instrument (2) having a drug solution reservoir part (62) that is configured to reserve a drug solution (M1);
an air vent needle tube (76) configured to be held by the main body part (71), configured to be connected to a drug solution container (4), and through which gas that is sent to the drug solution container (4) can pass;
a liquid supply needle tube (75) configured to be held by the main body part (71), configured to be connected to the drug solution container (4) and a filling port (62a), and through which the drug solution (M1) that is sent from the drug solution container (4) to the drug solution reservoir part (62) can pass, the filling port (62a) being provided on the drug solution reservoir part (62), the drug solution (M1) being injected from the filling port (62a); and
a suction channel (95) configured to communicate with a liquid supply port (62b) and communicate with a suction mechanism (13) that is configured to suck in gas within the drug solution reservoir part (62), the liquid supply port (62b) being provided on the drug solution reservoir part (62) to send the drug solution (M1).

## Patentansprüche

1. Fülleinheit (5) für eine Arzneimittellösung, umfassend:
ein Instrument (2) mit einem Vorratsbehälter für eine Arzneimittellösung, das einen Teil (62) mit einem Vorratsbehälter für eine Arzneimittellösung besitzt, der dazu ausgebildet ist, eine Arzneimittellösung (M1) zu bevorraten; und
einen Fülladapter (3), der dazu ausgebildet ist, lösbar an dem Instrument (2) mit einem Vorratsbehälter für eine Arzneimittellösung befestigt zu werden, wobei das Instrument (2) mit einem Vorratsbehälter für eine Arzneimittellösung Folgendes aufweist:
den Teil (62) mit einem Vorratsbehälter für eine Arzneimittellösung, der einen Füllstutzen (62a) besitzt, aus dem die Arzneimittellösung (M1) injiziert werden kann; und
ein äußeres Gehäuse (61), das dazu ausgebildet ist, den Teil (62) mit einem Vorratsbehälter für eine Arzneimittellösung aufzunehmen, und wobei der Fülladapter (3) Folgendes aufweist:
ein Nadelrohr (75) für die Flüssigkeitszufuhr, das dazu ausgebildet ist, mit dem Arzneimittellösungsbehälter (4) und dem Füllstutzen (62a) verbunden zu werden und durch das die Arzneimittellösung (M1) fließen kann, die von dem Arzneimittellösungsbehälter (4) zu dem Teil (62) mit einem Vorratsbehälter für eine Arzneimittellösung geschickt wird;
**dadurch gekennzeichnet, dass**
der Teil (62) mit einem Vorratsbehälter für eine Arzneimittellösung ferner einen Flüssigkeitszufuhrstutzen (62b) aufweist, der dazu ausgebildet ist, die eingefüllte Arzneimittellösung (M1) zu schicken; und
der Fülladapter (3) ferner Folgendes aufweist:
ein Entlüftungsnadelrohr (76), das dazu ausgebildet ist, mit einem Arzneimittellösungsbehälter (4) verbunden zu werden und durch das Gas, das zu dem Arzneimittellösungsbehälter (4) geschickt wird, strömen kann; und
einen Saugkanal (95), der dazu ausgebildet ist, mit dem Flüssigkeitszufuhrstutzen (62b) in Verbindung zu stehen, und dazu ausgebildet ist, mit einem Saugmechanismus (13) in Verbindung zu stehen, der dazu ausgebildet ist, Gas in dem Teil (62) mit einem Vorratsbehälter für eine Arzneimittellösung anzusaugen.

2. Fülleinheit (5) für eine Arzneimittellösung nach Anspruch 1, wobei
der Saugkanal (95) mit einem Entlüftungsfilter (77) versehen ist, der dazu ausgebildet ist, nur Gas hindurchströmen zu lassen, und dazu ausgebildet ist, die Arzneimittellösung (M1) nicht hindurchströmen zu lassen.

3. Fülleinheit (5) für eine Arzneimittellösung nach Anspruch 1 oder 2, wobei
der Flüssigkeitszufuhrstutzen (62b) vertikal über dem Füllstutzen (62a) angeordnet ist, wenn die Arzneimittellösung (M1) eingefüllt wird.

4. Fülleinheit (5) für eine Arzneimittellösung nach einem der Ansprüche 1 bis 3, wobei
der Fülladapter (3) einen Schutzmechanismus (78) besitzt, der dazu ausgebildet ist, eine Nadelspitze des Nadelrohrs (75) für die Flüssigkeitszufuhr auf einer Seite zu bedecken, die dazu ausgebildet ist, mit dem Flüssigkeitszufuhrstutzen (62b) verbunden zu werden, nachdem der Füllvorgang für die Arzneimittellösung (M1) beendet ist.

5. Fülleinheit (5) für eine Arzneimittellösung nach einem der Ansprüche 1 bis 4, wobei
der Fülladapter (3) ein Stützteil (72) besitzt, das dazu ausgebildet ist, eine Nadelspitze von jedem von dem Entlüftungsnadelrohr (76) und dem Nadelrohr (75) für die Flüssigkeitszufuhr auf einer Seite zu bedecken, die dazu ausgebildet ist, mit dem Arzneimittellösungsbehälter (4) verbunden zu werden, und dazu ausgebildet ist, den Arzneimittellösungsbehälter (4) zu stützen.

6. Instrument (2) mit einem Vorratsbehälter für eine Arzneimittellösung, umfassend:
einen Teil (62) mit einem Vorratsbehälter für eine Arzneimittellösung, der einen Füllstutzen (62a) besitzt, aus dem eine Arzneimittellösung (M1) injiziert werden kann, und einen Flüssigkeitszufuhrstutzen (62b), der dazu ausgebildet ist, die eingefüllte Arzneimittellösung (M1) zu schicken; und
ein äußeres Gehäuse (61), das dazu ausgebildet ist, den Teil (62) mit einem Vorratsbehälter für eine Arzneimittellösung aufzunehmen, wobei Gas in dem Teil (62) mit einem Vorratsbehälter für eine Arzneimittellösung aus dem Flüssigkeitszufuhrstutzen (62b) angesaugt wird, wenn die Arzneimittellösung (M1) eingefüllt wird.

7. Fülladapter (3), umfassend:
einen Hauptkörperteil (71), der dazu ausgebildet ist, lösbar an einem Instrument (2) mit einem Vorratsbehälter für eine Arzneimittellösung befestigt zu werden, der einen Teil (62) mit einem Vorratsbehälter für eine Arzneimittellösung besitzt, der dazu ausgebildet ist, eine Arzneimittellösung (M1) zu bevorraten;
ein Entlüftungsnadelrohr (76), das dazu ausgebildet ist, von dem Hauptkörperteil (71) gehalten zu werden, das dazu ausgebildet ist, mit einem Arzneimittellösungsbehälter (4) verbunden zu werden, und durch das Gas, das zu dem Arzneimittellösungsbehälter (4) geschickt wird, strömen kann;
ein Nadelrohr (75) für die Flüssigkeitszufuhr, das dazu ausgebildet ist, von dem Hauptkörperteil (71) gehalten zu werden, das dazu ausgebildet ist, mit dem Arzneimittellösungsbehälter (4) und einem Füllstutzen (62a) verbunden zu werden, und durch das die Arzneimittellösung (M1), die von dem Arzneimittellösungsbehälter (4) zu dem Teil (62) mit einem Vorratsbehälter für eine Arzneimittellösung geschickt wird, fließen kann, wobei der Füllstutzen (62a) an dem Teil (62) mit einem Vorratsbehälter für eine Arzneimittellösung vorgesehen ist, wobei die Arzneimittellösung (M1) aus dem Füllstutzen (62a) injiziert wird; und
einen Saugkanal (95), der dazu ausgebildet ist, mit einem Flüssigkeitszufuhrstutzen (62b) in Verbindung zu stehen und mit einem Saugmechanismus (13) in Verbindung zu stehen, der dazu ausgebildet ist, Gas in dem Teil (62) mit einem Vorratsbehälter für eine Arzneimittellösung anzusaugen, wobei der Flüssigkeitszufuhrstutzen (62b) an dem Teil (62) mit einem Vorratsbehälter für eine Arzneimittellösung vorgesehen ist, um die Arzneimittellösung (M1) zu schicken.

## Revendications

1. Unité de remplissage de solution médicamenteuse (5) comprenant :
un instrument à réservoir de solution médicamenteuse (2) ayant une partie de réservoir de solution médicamenteuse (62) qui est adaptée pour contenir une solution médicamenteuse (M1) ; et
un adaptateur de remplissage (3) adapté pour être monté de façon détachable sur l'instrument à réservoir de solution médicamenteuse (2), où
l'instrument à réservoir de solution médicamenteuse (2) comprend :
la partie de réservoir de solution médicamenteuse (62) qui comporte un orifice de remplissage (62a) à partir duquel la solution médicamenteuse (M1) peut être injectée ; et
un boîtier externe (61) qui est adapté pour loger la partie de réservoir de solution médicamenteuse (62), et
l'adaptateur de remplissage (3) comprend :
un tube à aiguille de distribution de liquide (75) adapté pour être relié au récipient de solution médicamenteuse (4) et à l'orifice de remplissage (62a) et par lequel peut passer la solution médicamenteuse (M1) qui est envoyée depuis le récipient de solution médicamenteuse (4) vers la partie de réservoir de solution médicamenteuse (62) ;
**caractérisée en ce que**
la partie de réservoir de solution médicamenteuse (62) comporte en outre un orifice de distribution de liquide (62b) qui est adapté pour envoyer la solution médicamenteuse introduite (M1) ; et
l'adaptateur de remplissage (3) comprend en outre :
un tube à aiguille de passage d'air (76) adapté pour être relié à un récipient de solution médicamenteuse (4) et par lequel du gaz qui est envoyé vers le récipient de solution médicamenteuse (4) peut passer ; et
un canal d'aspiration (95) adapté pour communiquer avec l'orifice de distribution de liquide (62b) et adapté pour communiquer avec un mécanisme d'aspiration (13) qui est conçu pour aspirer du gaz à l'intérieur de la partie de réservoir de solution médicamenteuse (62).

2. Unité de remplissage de solution médicamenteuse (5) selon la revendication 1, dans laquelle
le canal d'aspiration (95) est muni d'un filtre de passage d'air (77) qui est adapté pour ne permettre que le passage du gaz et qui est adapté pour ne pas permettre le passage de la solution médicamenteuse (M1).

3. Unité de remplissage de solution médicamenteuse (5) selon la revendication 1 ou 2, dans laquelle
l'orifice de distribution de liquide (62b) est disposé verticalement au-dessus de l'orifice de remplissage (62a) lorsque la solution médicamenteuse (M1) est introduite.

4. Unité de remplissage de solution médicamenteuse (5) selon l'une quelconque des revendications 1 à 3, dans laquelle
l'adaptateur de remplissage (3) comprend un mécanisme de protection (78) qui est adapté pour couvrir une extrémité d'aiguille du tube à aiguille de distribution de liquide (75) au niveau d'un côté adapté pour être relié à l'orifice de distribution de liquide (62b) une fois que l'opération de remplissage de la solution médicamenteuse (M1) est terminée.

5. Unité de remplissage de solution médicamenteuse (5) selon l'une quelconque des revendications 1 à 4, dans laquelle
l'adaptateur de remplissage (3) comprend une partie de support (72) qui est adaptée pour couvrir une extrémité d'aiguille de chacun du tube à aiguille de passage d'air (76) et du tube à aiguille de distribution de liquide (75) au niveau d'un côté adapté pour être relié au récipient de solution médicamenteuse (4), et est conçue pour supporter le récipient de solution médicamenteuse (4).

6. Instrument à réservoir de solution médicamenteuse (2) comprenant :
une partie de réservoir de solution médicamenteuse (62) ayant un orifice de remplissage (62a) à partir duquel une solution médicamenteuse (M1) peut être injectée et un orifice de distribution de liquide (62b) qui est adapté pour envoyer la solution médicamenteuse introduite (M1) ; et
un boîtier externe (61) qui est adapté pour loger la partie de réservoir de solution médicamenteuse (62), où
le gaz à l'intérieur de la partie de réservoir de solution médicamenteuse (62) est aspiré depuis l'orifice de distribution de liquide (62b) lorsque la solution médicamenteuse (M1) est introduite.

7. Adaptateur de remplissage (3) comprenant :
une partie de corps principal (71) adaptée pour être montée de façon détachable sur un instrument à réservoir de solution médicamenteuse (2) comprenant une partie de réservoir de solution médicamenteuse (62) qui est adaptée pour contenir une solution médicamenteuse (M1) ;
un tube à aiguille de passage d'air (76) adapté pour être maintenu par la partie de corps principal (71), conçu pour être relié à un récipient de solution médicamenteuse (4), et par lequel du gaz qui est envoyé vers le récipient de solution médicamenteuse (4) peut passer ;
un tube à aiguille de distribution de liquide (75) adapté pour être maintenu par la partie de corps principal (71), adapté pour être relié au récipient de solution médicamenteuse (4) et à un orifice de remplissage (62a), et par lequel la solution médicamenteuse (M1) qui est envoyée depuis le récipient de solution médicamenteuse (4) vers la partie de réservoir de solution médicamenteuse (62) peut passer, la solution médicamenteuse (M1) étant injectée depuis l'orifice de remplissage (62a) ; et
un canal d'aspiration (95) adapté pour communiquer avec un orifice de distribution de liquide (62b) et pour communiquer avec un mécanisme d'aspiration (13) qui est conçu pour aspirer du gaz à l'intérieur de la partie de réservoir de solution médicamenteuse (62), l'orifice de distribution de liquide (62b) étant placé sur la partie de réservoir de solution médicamenteuse (62) pour envoyer la solution médicamenteuse (M1).
